# EUROPEAN PATENT APPLICATION

(11) **EP 1 767 257 A1**
(43) Date of publication of application: **28.03.2007**
(21) Application number: 05447216.2
(22) Date of filing: 22.09.2005
(51) Int. Cl.: B01D 1/28, B01D 1/26, C02F 1/16, C02F 1/06

(54) **Installation for the treatment of liquid**

(71) Applicant: VLAAMSE INSTELLING VOOR TECHNOLOGISCH ONDERZOEK (VITO), 2400 Mol (BE)
(72) Inventor: Brauns, Etienne, 2400 Mol (BE); Liekens, Johan, 2250 Olen (BE); Van Bael, Johan, 2300 Turnhout (BE)
(74) Representative: Van Malderen, Joëlle

(57) **Abstract**

The present invention is related to an installation for treating a liquid, in particular waste water, comprising an evaporator system (1) of the Mechanical Vapour Recompression type, said system comprising a primary evaporator (2) and a compressor means (4), the installation further comprising at least one further evaporator or dryer (8) arranged to further treat a concentrated liquid supply produced by said MVR system,
characterized in that the installation further comprises a combined heat and power (CHP) system, arranged to simultaneously :
- power the compressor means of said MVR system, and
- supply heat for the operation of the further evaporator(s) or dryer(s).

## Description

### Field of the Invention

The present invention is related to liquid treatment systems, in particular systems comprising evaporators, consisting of a primary Mechanical Vapor Recompression (MVR) based evaporator and one or more further (secondary, tertiary, ...) evaporators.

### State of the art

The concept of an evaporator based on the principle of mechanical vapor recompression (MVR) is well known, as illustrated for example in US4303468. In a MVR based evaporator the vapor from the evaporator effect (possibly multiple effects) is recompressed in order to increase the vapor's temperature. In this way the recompressed vapor can be reused as a heating medium in the MVR based evaporator effect during which the vapor condenses and releases its heat of condensation. The MVR based evaporator therefore functions as a heat pump. The latent heat of evaporation is regained as the heat of condensation. When compared to the latent heat of evaporation the MVR based evaporator therefore requires only a small amount of energy (typically about 2 % of the latent heat of evaporation). However, a MVR based falling film evaporator is restricted to evaporating up to a dry solids content in the concentrate of about 15 to 20 %.

This limitation is a result of the increasing concentrate viscosity with increasing dry solids content. When using for example a falling film evaporator effect, the increased concentrate viscosity will finally result in a malfunctioning of the falling film. Therefore the concentrate effluent of the MVR based evaporator is mostly treated further with one or more additional evaporators. These succeeding complementary evaporators are often a forced loop evaporator and thermal evaporation heat is being supplied as e.g. steam within a heat exchanger. The complementary evaporators are able to produce a concentrate with a typical dry solids content in the concentrate of 50 - 80 %. As an indication, the volume of a typical feed with a dry solids content of 1 % , can be reduced in the MVR based evaporator with a factor of about 15 (100 m³ of feed will then produce 6.67 m³ of concentrate and 93.33 m³ of purified condensate) while the one or more additional evaporators will further reduce the 6.67 m³ of MVR concentrate to a concentrate with a typical dry solids content of 50 % (concentrate end volume of 2 m³).

The second (and possible further) evaporator(s) which treats the concentrate from the MVR based evaporator consumes a considerable amount of heat energy. This energy consumption in the second (and possible further) evaporator(s) constitutes an important part of the total cost of running the total evaporation installation.

### Aim of the invention

The present invention aims at the further reduction of the evaporation primary energy consumption in an installation with a main MVR evaporator and one or more secondary evaporators.

### Summary of the invention

The present invention is related to an installation such as described in the appended claims. It concerns an installation wherein a Combined Heat and Power installation is introduced in an evaporation installation, consisting of an MVR based primary (e.g. falling film) evaporator and one ore more succeeding (e.g. forced loop) evaporators.

The principle of Combined Heat and Power, or CHP as it is more commonly referred to, is the simultaneous generation of usable heat and power (usually electricity) in a single process. For example, CHP systems may comprise one of the following installations : gasturbines, microturbines, reciprocating engines, Stirling engines, fuel cells, steam turbines, ....

CHP is sometimes referred to as co-generation or cogen. In typical conventional power generation, much of the total energy input is wasted. Combined Heat and Power or co-generation, where the heat produced in electricity generation is put to good use, can reach efficiencies in excess of 85%. CHP can provide a secure and highly efficient method of generating power (electricity) and heat at the point of use. Due to utilization of heat from power generation and the avoidance of transmission losses because power is generated on site, CHP achieves a significant reduction in primary energy usage compared with central power stations and ordinary boilers. Typically a good CHP scheme can realise a reduction in total primary energy requirements of 20% / 25% compared to the separate energy system it replaces. For an energy intensive business this can represent a very substantial saving.

### Short description of the figures

Figure 1 is a schematic overview of an installation according to a first embodiment of the invention.

Figure 2 is a schematic overview of an installation according to a second embodiment of the invention.

### Detailed description of the invention.

The present invention is related to an installation wherein a CHP installation is combined with an evaporation installation, based on a primary MVR based evaporator (called evaporator A) and one or more succeeding evaporators (called evaporator B), which treats the concentrate from evaporator A. In an installation according to the invention, the power (or part of the power) generated by the CHP is used for driving the compressor means coupled to the evaporator A. The heat (or part of the heat) generated by the CHP is used for driving, i.e. for supplying heat to the evaporator B. In the ideal configuration, the power and heat production of the CHP matches the power demand of evaporator A and the heat demand of evaporator B. It has been found that for most CHP intallations, in particular for a small gasturbine (upto 15MW) or small reciprocating engine (< 0,5 MWe), this ratio is fairly well defined to be at 1/2 (power/heat), which matches well with the ratio of the power demand of many existing evaporation installations with an MVR based evaporator as the primary evaporator, combined with further (secundary/tertiary) evaporators. This is why the present invention provides an effective solution to the prior art problem descrbibed above. The invention is however not restricted to installations requiring the ½ ratio. A steam turbine can for example be applied in case of a smaller power/heat ratio (heat required is more than double the power).

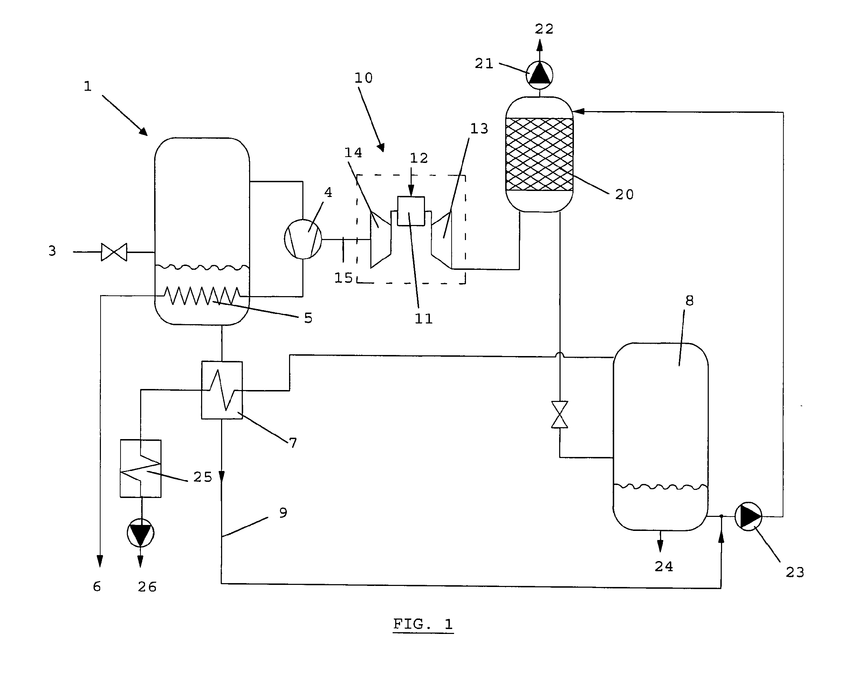

Figure 1 shows a schematic view of an installation according to a preferred embodiment of the invention. It comprises an MVR type evaporation system 1, comprising an evaporator vessel 2, supplied with a liquid to be treated (e.g. waste water) through a liquid supply 3. The figure is merely a representation of the principle of an installation of the invention, and its components may take on equivalent forms to the ones which are depicted. For example, the 'vessel' may be a liquid tank as represented in the drawing, or in the case of a falling film evaporator, it is constituted by the inside space of the tubes within the vertical bundle of evaporation tubes. Also other forms of evaporation or drying technologies can be used. For example, the MVR based primary evaporation system can consist of more than one evaporator. In such case the "vessel" consists of two (or more) evaporators (e.g. falling film evaporator) in series in such a way that the first evaporating effect is designed according to an optimal maximum concentration of its concentrate when operated in feed-and-bleed mode. The second evaporator in the primary MVR based evaporation system than further treats the concentrate from the first evaporator in the primary MVR based evaporation system. By having more than one effect in the primary MVR based evaporation system, the overall efficiency of the MVR based evaporation system is higher when compared to only a single effect.

The liquid is evaporated to form a vapour which is recompressed in compressor means 4, and thus reaches a higher temperature, after which it heats the supplied liquid in heat exchanger 5. During this heat exchange, the vapour condenses (which takes place at the outer surface of the vertical tubes in the case of a falling film evaporator) and is recuperated as purified liquid at 6. The MVR-concentrate 9 can be pre-heated in a further heat exchanger 7 and sent towards the secondary evaporator 8.

Typical to the installation of the invention, a CHP installation is present. In figure 1 this is a gasturbine 10, consisting of a combustion chamber 11 to which fuel 12 is supplied, a compressor 13 for compressing combustion air, and a turbine 14 for generating mechanical power. The latter is used for powering the compressor means of the MVR. This can be done by a direct mechanical coupling 15 between the shafts of these installations, or indirectly, in which case the turbine is coupled to a generator which feeds the electricity grid, from which the compressor in turn obtains its energy. Alternatively, the electricity generated by the CHP (with a frequency that can differ from the electricity grid) can be arranged to directly supply a special electromotor (e.g. based on DC or HF-AC) driving the compressor. By this approach, electricity losses from an electricity converter can be avoided.

The exhaust gases from the gas turbine are fed to a heat exchanger 20, wherein these gases are cooled and released, possibly through an exhaust fan 21, to the stack 22. The concentrate coming from the MVR 1 is pumped (at 23) to the heat exchanger and thereby heated and fed to the secondary evaporator 8, from which the end-concentrate 24 is obtained. In the embodiment shown, the secondary evaporator is arranged as a forced loop evaporator as the liquid in the evaporator 8 is constantly recirculated via the heat exchanger. In this configuration, the heat exchanger 20 heats up the supply of MVR concentrate, as well as the the liquid in the secondary evaporate which is being re-circulated. Other arrangements might be used for the secondary or further evaporator. These arangements may be any evaporation or drying technology (e.g. spray drying in counterflow with exhaust gases, vacuümdrying via an intermediate steam or hot waterloop heated by the cogeneration unit, ...).

The vapour produced in the secondary evaporator can be used, possibly after compression, to preheat the concentrate coming from the MVR, in heat exchanger 7. The vapour may then be further cooled to the point of condensing, in condensor 25, after which it is obtained as purified liquid 26. Heat recuperation can thus be optimised by preheating (at 7) the concentrate of the MVR and cooling (at 7 and 25) the purified liquid vapor from the succeeding evaporator(s) . The possibilities of heat recuperation differ from case to case (components to be purified as well as their concentrations) and this invention is not restricted to the installations shown in the figures.

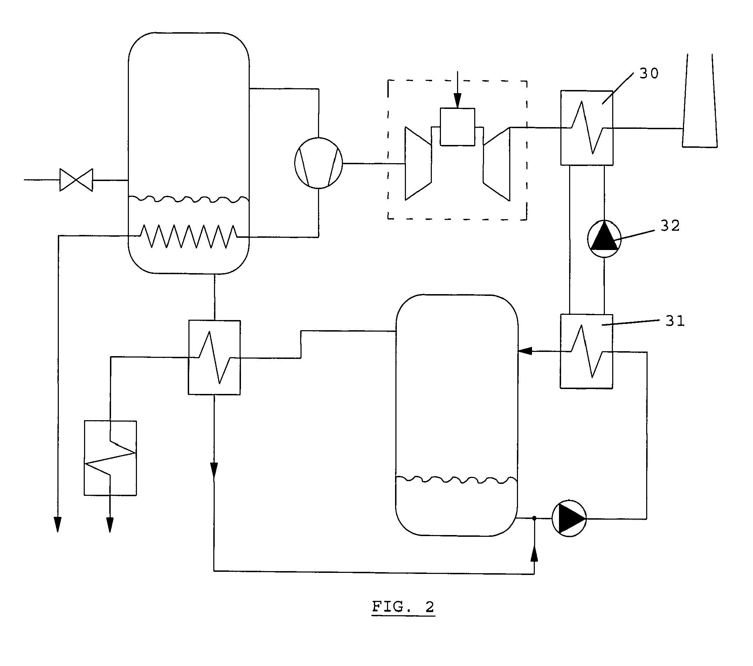

Figure 2 shows an alternative, wherein the heat exchanger 20 takes on the form of a 'run-around-loop', consisting of two separate heat exchangers 30 and 31 and a cycle in which a separate fluid is pumped around by pump 32, to subsequently take up heat in heat exchanger 30 and release heat to heat up the concentrate, in heat exchanger 31.

Another alternative is the heating of the concentrate by direct contact with the exhaust gases coming from the gas turbine or other CHP-technology.

In stead of the gas turbine 10, any installation may be used which is capable of producing mechanical energy and heat simultaneously. The preferred cases are however gas turbines and reciprocating engines, given that they have a power/heat ratio which coincides well with the required power and heat ratio of an MVR installation with one or more further evaporators. In the case of a reciprocating engine, all or a part of the heat produced by the engine can be recuperated according to the temperature requirements of the total purification process. The heat in the exhaust gasses of the engine can be used for direct or indirect heat exchange with the concentrate in the succeeding evaporator(s).
The CHP can also involve a steam turbine. This may depend on the type of secondary and further evaporator(s). If these evaporators require a larger amount of heat compared to the power demand of the MVR, then a steam turbine may be a suitable choice for the CHP. This might be the case for example when the share of the MVR-evaporation in the total evaporation is small because of the specific proces requirements.

As a fuel source for the CHP, besides conventional fuels the usage of biomass (e.g. biogas) could also be considered. In the case of the availability of an anaerobic digester when treating waste waters, the biogas from this digester is a candidate as a fuel gas. This would further decrease the consumption of fossil fuels. Advantageously, the biogas is obtained from the concentrate itself, since the concentration is higher and therefore possibly more adapted as a feed for a digester.

The combination of the evaporators A and B with the CHP would significantly promote the use of the MVR evaporation technology when treating waste waters. As an example, waste waters from a classic biological water treatment system typically have a dry solids content of 1 % and are therefore suited for an evaporation treatment involving an evaporator A (MVR based) and a succeeding evaporator B. As explained, the evaporation through evaporator A and B enables to reduce the volume of the waste water by a factor of typically 50. In addition, the major part of the obtained condensate can be directly used in most cases as process water. Moreover, by further treating the remaining small volume of concentrate from evaporator B in a small tertiary evaporator, it is possible in principle to convert it to a dry solid waste. The CHP could also provide the heat energy needed by this tertiary evaporator. In this way a near-closed process water network could be obtained (depending on the process conditions). Also the problem of the concentrate disposal (contamination of surface water) could be resolved in this way since a dry solid waste can be treated in a more specific way by specialized waste treatment companies or even, when containing an important organic fraction, be used as a (bio-)fuel. A zero waste disposal on surface water could be the result, protecting in an important way the environment. The re-use of the condensate as process water also protects the environment in an additional way since e.g. valuable (deep) ground water resources are less solicited in this way.

The proposed combination allows for the production of mechanical/electrical power by the CHP needed by the MVR and the production of heat by the CHP needed by the secondary evaporator (or tertiary evaporator when envisaging a dry solids waste fraction when treating waste waters). This novel combination enables to further optimize the energy consumption of the evaporators installation and therefore initiates a reduction in fossil fuel consumption, resulting from the local production of the power and the direct useful usage of the heat in the secondary (tertiary) evaporator(s) (cogeneration effect). Additionally this invokes a reduction of emissions (such as CO₂) and a reduction in energy costs. The economical benefit could promote the application of the novel method within the treatment of waste water, the recovery of process water from the condensate produced in the primary MVR based evaporator and the production of a solid waste fraction. In this way a near closed process water loop could be obtained in principle with :
- a zero waste disposal, thus totally avoiding surface water contamination.
- the protection of valuable water resources

Although the text above describes the use of this invention for waste water processing, the overall principle can also be applied for purification purposes of other fluid mixtures and contaminants. The possibilities of the technique discribed in this invention for the particular stream must be investigated.

## Claims

1. An installation for treating a liquid, in particular waste water, comprising an evaporator system (1) of the Mechanical Vapour Recompression type, said system comprising a primary evaporator (2) and a compressor means (4), the installation further comprising at least one further evaporator or dryer (8) arranged to further treat a concentrated liquid supply produced by said MVR system,
**characterized in that** the installation further comprises a combined heat and power (CHP) system, arranged to simultaneously :
- power the compressor means of said MVR system, and
- supply heat for the operation of the further evaporator(s) or dryer(s).

2. The installation according to claim 1, wherein said CHP system comprises a gasturbine (10).

3. The installation according to claim 1, wherein said CHP system comprises a reciprocating engine.

4. The installation according to claim 1, wherein said CHP system comprises a steam turbine.

5. The installation according to any one of claims 1 to 4, wherein the further evaporator(s) or dryer(s) (8) is(are) arranged in a forced loop.

6. The installation according to claim 5, comprising a heat exchanger means (20,30,31) arranged for heating up said concentrated liquid supply as well as the liquid circulating in said forced loop.

7. The installation according to any one of the preceding claims, wherein said CHP system is arranged to produce electrical energy and supply that energy to an electricity grid, and wherein said compressor (4) is powered by electricity from said grid.

8. The installation according to any one of claims 1 to 6, wherein said CHP system is arranged to directly power an electric motor, said motor being arranged to power said compressor (4).

9. The installation according to any one of claims 1 to 6, wherein said CHP system is arranged to power directly, by a mechanical coupling, said compressor (4).

10. The installation according to any one of the preceding claims, arranged so that said CHP system is fueled by biogas, produced from said liquid.
